# EUROPEAN PATENT SPECIFICATION

(11) **EP 0 320 130 B1**
(45) Date of publication and mention of the grant of the patent: **29.06.1994**
(21) Application number: 88310833.4
(22) Date of filing: 16.11.1988
(51) Int. Cl.: A01N 63/04, A61K 35/70, C12N 1/14, C12P 1/02

(54) **Fungicidal active principles, fungicidal compositions, methods of combating fungi, processes of preparing fungicidal active principles, a fungus and fungicidal additives**
Grundbestandteile mit fungizider Wirkung, fungizide Zusammensetzungen, Verfahren zur Pilzbekämpfung, Verfahren zur Herstellung von Grundbestandteilen mit fungizider Wirkung, ein Pilz und fungizide Zusatzmittel
Principes actifs comme fongicides, compositions fongicides, procédés de lutte contre les champignons, procédés de préparation de principes actifs comme fongicides, un champignon et additifs fongicides

(30) Priority: 17.11.1987 DK 6046/87
(43) Date of publication of application: 14.06.1989
(73) Proprietor: NOVO NORDISK A/S, 2880 Bagsvaerd (DK)
(72) Inventor: Nielsen, Ruby Ione, DK-3520 Farum (DK)
(74) Representative: Jensen, Bo Hammer

(56) References cited:
- EP-A- 0 207 653

## Description

### FIELD OF THE INVENTION

This invention relates to a biochemically active principle obtainable by cultivation of fungi of the genus Phaeoramularia fungicidal compositions comprising a fungicidally effective amount of said principle, agronomically or physiologically compatible diluent and/or carrier means, and optionally other active materials; and the use of said principle and fungicidal compositions for combating fungi in plants or animals, including mammals, and as a preservative in edibles, paints and on timber.

### PRIOR ART

Fungi are at present controlled in plants by applying synthetic inorganic or organic fungicides to the area to be treated.

Such fungicides are costly and energy consuming in their production, and often they are not very effective in combating the fungi. This may often be due to climatic or environmental conditions prevailing in the area or time of applying. Also, the use of a fungicide over a prolonged period of time tends to select for fungi which are tolerant to the fungicide.

Most of the known fungicides being alien to the ecosystems wherein they are spread also tend to accumulate and eventually exhibit undesired side effects.

For these and other reasons it is desirable to develop biological fungicides that are easily digested in nature and do not consume the large amount of energy required for the chemical synthesis of most known fungicides.

Through the work of the inventor it was surprisingly found that fungi belonging to the genus Phaeoramularia exhibit interesting properties in this respect.

The genus Cercospora is known worldwide as the cause of leaf spot diseases on a large number of hosts of different genera. Some species of Cercospora are known to produce different metabolites e.g.
C. beticola produces fulvic acid (Sakaki T., Ichihara A., Sakamura S. Isolation of fulvic acid from Cercospora beticola. Agric.Biol.Chem. 45(5): 1275-1276 (1981)) and a yellow phytotoxin (Martin S.S., Steinkamp M.P. The yellow phytotoxins of Cercospora beticola. Proc.Int.Bot.Congr. 13: 296 (1981)).
C. kikuchii produces cercosporin (Matsueda S., Takagaki K., Shimoyama M., and Shiota A. Studies on fungal products. V. Antimicrobial aspects of Quinone derivatives. Yakugaku Zasshi 100(9):900-902 (1980)) and ergosterol-5,8-peroxide (Matsueda S., Shimoyama M., Imaizumi T., Tsushima Y., Studies on fungal products 4. Biological effects of ergosterol-5,8- peroxide. Yakugaku Zasshi 102(4): 347-351 (1982)).

Matsueda et al. found cercosporin to inhibit the growth of Staphylococcus aureus, Bacillus subtilis, Pseudomonas Aeruginosa, E. coli and Candida japonica. Cercosporin has been isolated as red crystals, Pfizer Handbook of Microbial Metabolites McGraw-Hill Book Co.Inc. N.Y. Toronto London from Shimpei Kuyama and Teiichi Tamura. J.Am.Chem.Soc. 79: 5725-5726 (1957).
C. arachidicola produces an anthraquinonoid metabolite (Stoessl A., Stothers J.B., Minor anthraquinonoid metabolites of Cercospora arachidicola. Can.J.Chem. 63(6): 1258-1262 (1985)), and
C. cruenta produces metabolites structurally related to abscisic acid (Takayuki Oritani, Kyohei Yamashita. Isolation and structure of 4'-hydroxy-γ-ionylidene acetic acids from Cercospora cruenta, a fungus producing (+) - Abscisic acid. Agric.Biol.Chem. 51(1): 275-278 (1987)), such as mutasteine (Japanese Patent No. 98920 issued 861027)
C. rosicola produces abscicic acid (Japanese Patent Application No. 192674 filed 8211102).
Phaeoramularia (then Cercospora) fusimaculans has been isolated from a number of grasses (Ellis M.B., More dematiaceous Hyphomycetes. Commonwealth Mycological Institute, Kew, Surrey, England (1976)) and has been reported to cause foliar disease of sorghum spp. (Wall G.C., Mughogho L.K., Frederiksen R.A., Odvody G.N. Foliar disease of sorghum spp. caused by Cercospora fusimaculans. Plant Dis. 71(8): 759-760 (1987)) and Bajra Pennisetum typhoides (Patel K.S. A Cercospora fusimaculans leaf spot disease of Bajra Pennisetum typhoides in Gujarat. Curr.Sci. (Bangalore) 42(1): 34). So far there has been no recording of P. fusimaculans producing a fungicidally active principle.

### SUMMARY OF THE INVENTION

As alluded to above the present inventor surprisingly found that fungi belonging to the genus Phaeoramularia had interesting fungicidal properties. Consequently this invention relates to a biochemically active principle obtainable by cultivation of Phaeoramularia fusimaculans (Atk), Liu & Guo CBS 616.87, which principle has been found to exhibit a unique fungicidal activity.

Accordingly the invention in a second aspect relates to a fungicidal composition comprising an effective amount of a fungicidally active principle obtainable by cultivation of a fungus of the genus Phaeoramularia fusimaculans (Atk), Liu & Guo CBS 616.87, and an agrochemically and/or physiologically compatible carrier or diluent.

In a further aspect the invention relates to a fungicidal composition comprising a suspension of an effective number of spores or mycelium from a fungus of Phaeoramularia fusimaculans (Atk), Liu & Guo CBS 616.87, in a compatible medium.

In another aspect the present invention relates to a method of combating fungi in plants, wherein an effective amount of a fungicidally active principle obtainable by cultivation of a fungus of the genus Phaeoramularia fusimaculans (Atk), Liu & Guo CBS 616.87, is applied to a region to be treated. In this aspect of the invention is also included combating or controlling fungi in edibles, timber, paint and the like, including growth media for microorganisms.

In yet a fifth aspect this invention relates to the use of a fungicidally active principle obtainable by culturing a fungus belonging to the genus Phaeoramularia fusimaculans (Atk), Liu & Guo CBS 616.87, in controlling fungi in plants.

A sixth aspect of the invention concerns a process for the production of a fungicidally active principle by cultivating a fungus of the genus Phaeoramularia fusimaculans (Atk), Liu & Guo CBS 616.87, and recovering said active principle.

### DEPOSITION OF MICROORGANISMS

A strain of the genus Phaeoramularia, Phaeoramularia fusimaculans (Atk), Liu & Guo, is on deposit at the Centraalbureau voor Schimmelcultures (CBS), P.0. Box 273, NL-3740 AB BAARN, Holland for the purposes of patent procedure on the date indicated below. CBS being an international depository authorized under the Budapest Treaty affords permanence of the deposit in accordance with rule 9, of said Treaty.
Deposit date: 22 October 1987
Depositors ref. Cercospora fusimaculans Atk
CBS designation: CBS 616.87

### DETAILED DESCRIPTION OF THE INVENTION

As indicated this invention in its first aspect relates to a fungicidally active principle obtainable by cultivation of CBS 616.87.

In its second aspect the invention relates to a fungicidal composition comprising an effective amount of a fungicidally active principle according to the preceding paragraph and an agrochemically and/or physiologically compatible carrier or diluent.

Depending on the circumstances such as the crop wherein fungi are to be combated, the environmental conditions or other factors, the composition of the invention in addition to said fungicidally active principle may also contain other active ingredients such as other biocides, pesticides, herbicides, insecticides, nematocides, acaricides or plant nutrients or fertilizers.

For combating fungi in animals the composition of the invention would usually comprise said active principle alone with a physiologically compatible carrier or diluent, but it may also be combined with other active ingredients such as an antibioticum.

In a special aspect of the invention there is provided a composition comprising a suspension of an effective number of viable units (spores or mycelium) of Phaeoramularia fusimaculans (Atk), Liu & Guo, or a mutant or genetically engineered transformant having the same characteristic ability to produce said active principle suspended in a compatible medium.

Said composition is conveniently made by using a liquid growth medium for producing the fungus, which medium subsequently is diluted to a suitable number of viable units (spores and/or mycelium) per ml, typically in the range from 10² to 10¹⁰, and preferably from about 10⁴ to about 10⁶.

Subsequently said suspension is applied to the area to be treated. The amount applied to said area may vary depending on the same circumstances as alluded to above.

A fungicidal composition according to the invention having the fungicidally active principle as its active ingredient may for agronomical and/or horticultural applications be formulated by mixing the active principle with suitable inert and compatible carriers or diluents to obtain a composition of the type generally used in agricultural compositions such as a wettable powder, an emulsifiable concentrate, a granular formulation, a water soluble powder, an alginate, a xanthan gum and/or an aerosol. As solid carriers bentonite, diatomaceous earth, apatite, gypsum, talc, pyrophyllite, vermiculite, ground shells, and clay may be mentioned. A surface active agent may also be added with the purpose of producing a homogeneous and stable formulation.

The diluent or carrier in the compositions of the invention can as indicated be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyaryl sulphonates such as alkyl-benzene sulphonates or lower alkylnaphthalene sulphonates, e.g. butyl-napthhalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- of alkenyl-sustituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene oxide, acetylenic glycols such as 2,4,7,9-tetraethyl-5-decyn-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, a dispersion, an aqueous emulsion, a dusting powder, a seed dressing, a dispersible powder, an emulsifiable concentrate or granules. Moreover, it can be in a suitable form for direct application or as a concentrate or primary composition which requires dilution with a suitable quantity of water or other diluent before application.

An emulsifiable concentrate comprises the active ingredient dissolved in a water-immiscible solvent which is formed into an emulsion with water in the presence of an emulsifying agent.

A dusting powder comprises the active ingredient intimately mixed and ground with a solid pulverulent diluent, for example, kaolin.

A granular solid comprises the active ingredient associated with similar diluents to those which may be employed in dusting powders, but the mixture is granulated by known methods. Alternatively it comprises the active ingredient absorbed or adsorbed on a pre-granular diluent for example, Fuller's earth, attapulgite or limestone grit.

Wettable powders, granules or grains usually comprise the active ingredient in admixture with a suitable surfactant and an inert powder diluent such as china clay.

Another suitable concentrate is a flowable suspension concentrate which is formed by grinding the active ingredient with water or other liquid, a wetting agent and suspending agent.

For use in combating fungi in animals including mammals said active principle may be formulated by mixing said principle with suitable inert and compatible carriers known in the art for use in topical formulations.

The concentration of the active principle described herein in the compositions of the invention may vary within a wide range depending on the type of formulation and the field of application.

In connection herewith it is mentioned that tests mentioned later have shown the active principle to be efficient to control fungi attacking mammals in concentrations from 0.5 »g/ml to 5 »g/ml and it is consequently contemplated that the active principle may be applied in concentrations ranging from about 0.01 »g/ml to 10 »g/ml for use in controlling fungi in animals.

In its third aspect the invention relates to a method of combating fungi in plants, wherein an effective amount of a fungicidally active principle obtainable by cultivation of CBS 616.87 is applied to a region to be treated.

In connection with this aspect the compositions of the invention may for agronomical or horticultural uses be applied to a region to be treated either directly to the soil as a pre-emergence treatment or to the foliage or fruits of the plants as a post-emergence treatment. Depending on the crop and circumstances the treatment may be postponed until seeds or fruits appear on the plants, wherein fungi are to be controlled.

The active preparation or the compositions of the invention can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth as this is the time when the plant can be most severely damaged. The spray or dust can conveniently contain a pre- or post-emergence herbicide if this is thought necessary.

Sometimes, it is practicable to treat the roots of a plant before or during planting, for example, by dipping the roots in a suitable liquid or solid composition. When the active preparation of the invention is applied directly to the plant a suitable rate of application is from 0.001 to 100 kg per hectare, preferably from 0.05 to 5 kg per hectare.

In the method of the invention the active preparation of the invention alone or in combination with a conventional biocide can also be applied to seeds or habitat. Thus the preparation can be applied directly to the soil before, at or after drilling so that the presence of active ingredient in the soil can control the growth of fungi which may attack seeds.

When the soil is treated directly the active preparation alone or in admixture with the conventional biocide can be applied in any manner which allows it to be intimately mixed with the soil such as by spraying, by broadcasting a solid form of granules, or by applying the active ingredient at the same time as drilling by inserting it in the same drill as the seeds. A suitable application rate is within the range of from 0.01 to 20 kg per hectare, more preferably from 0.05 to 10 kg per hectare.

For veterinary and medicinal uses the composition of the invention may be applied to a region to be treated when the diagnosis has been established by the veterinarian or physician.

The compositions may be applied in amounts corresponding to from about 1 g to about 100 kg fungicidally active principle per hectare.

Although the present invention has been described in detail in connection with controlling fungi in animals and plants, it is also anticipated that the fungicidally active principle may be used for the preservation of wood by adding said principle to wood preservation and/or impregnation compositions. Also, the active principle of the invention may be useful as a fungicide and preservant in paints - both to prevent growth in the paint during storage, and growth on the painted object such as the plastered surface of a house.

Further the fungicidally active principle of the invention may due to its low toxicity be used for the preservation of edible goods such as jams, marmelade, or other such items where the principle may be added subsequent to any cooking process.

Also the fungicidally active principle of the invention may be used as fungicidal additive to growth media for various microorganisms such as E. coli, B. subtilis, Pseudomonas aerugenosa, and S. aureus.

### THE FUNGICIDALLY ACTIVE PRINCIPLE

The fungicidally active principle of the invention may be produced extracellularly in both surface and submerged substrates containing different carbohydrates and nitrogen sources. When applied in the crude state as it is produced in shake flasks, it has no apparent effect on the growth of
E. coli, Pseudomonas aeruginosa, Bacillus subtilis, Torulopsis ernobii, Rhodotorula ruda, Rhodotorula gracilis, Hansenula holstii, Candida albicans, Penicillium funiculosum, Aspergillus fumigatus, Aspergillus aculeatus, Aspergillus phoenicis, Aspergillus awamori,and Staphylococcus aureus.
It has been found to have an inhibitory effect on the growth of fungi of the following genera
Botrytis, Rhizomucor, Pyricularia, Fusarium, Penicillium, and especially on Botrytis cinerea, Rhizomucor miehei, Pyricularia oryzae, Penicillium digitatum, Cercospora traversiana, Cercospora sorghii, Cercospora sesami, Cercospora scirpicola, Cercospora kikuchii, Cercospora haji, Cercospora beticola, Sclerotinia fructicola, Aspergillus oryzae, Fusarium culmorum, Fusarium decemcellulase, Fusarium acuminatum, Fusarium moniliforme, Fusarium sporotrichioides, Fusarium poae, Fusarium fugikurai, Fusarium longipes, Rhizopus oligosporus, Trichophyton, Microsporum, Epidermaphyton, Pichia sp., Torulopsis spherica, Rhodotorula aurantiaca, Rhodotorula glutinis, Rhodotorula minuta, Candida albicans, Trichoderma viride, Drechslera australiensis, Gibberella fugikuri, Phoma herbarum, Pycnoporus sangiuneus, Penicillium notatum, Penicillium oxalicum, Penicillium crustosum, Penicillium implicatum, Penicillium lividum, Penicillium cyclopium, Penicillium digitatum, Penicillium italicum, Aspergillus cervinus, Aspergillus cremens and many others.

Above it was indicated that Matsueda et al. 1980 found that cercosporin a compound isolated from C. kikuchii had an inhibitory effect on certain microorganisms. From the preceding paragraph it is obvious that the active principle of the invention is different from cercosporin. Also, a red pigment produced by CBS 616.87, which could contain cercosporin, could readily be separated from the active principle of this invention without affecting the activity of said principle. Said pigment does not exhibit any inhibition on the growth of Botrytis cinerea.

The principle is active over a wide range of pHs and temperatures but is inactivated when boiled for about one minute. There is no apparent loss of activity when stored as culture broth in the refrigerator for 3 months.

It shows no indication of mutagenic activity when tested in the presence and absence of the S-9 metabolic activation system (Ames test). Culture broth containing the fungicide and viable spores showed no effect on mice, intraperitoneally applied at the rate of 20 ml per kg body weight. This contained 3 X 10⁸ viable counts.

### THE FUNGICIDE PRODUCING STRAINS

The fungi which produce the active principle of the invention have been identified as belonging to the genus Phaeoramularia.

The colonies are mostly hypophyllous. Conidiophores fasciculate, pale olivaceous brown, with small scars. Conidia often catenate, hyaline, nearly always 3-4 septate, 15-70 x 2-2.5 »m.

CBS 616.87 was isolated from leaves of Panicum maximum collected in St. Andrews, Jamaica in Dec. 1986. It has been identified as Phaeoramularia fusimaculans. At the same time a number of Phaeoramularia producing a fungicidally active principle were isolated from leaves of P. maximum, and in August 1988 Phaeoramularia also producing the same similar activity were isolated from leaves of P. maximum collected in St. Catherine, Jamaica. The species can be isolated from leaves of many different grasses (Ellis supra).

### CULTIVATION OF STRAINS

The fungicide producing strain may be grown on agar slants containing the following ingredients in grams/litre: yeast extract 4.0, potassium dihydrogen phosphate 1.0, magnesium sulphate heptahydrate 0.1, glucose 15 and BactoTM (Difco Laboratories, Detroit, USA) agar 20. This substrate is autoclaved at 121°C for 20 or 40 minutes and will, hereinafter, be referred to as YPG agar. Slants contain 12 ml YPG agar and after inoculation they are incubated at 20-25°C for 7 days or longer.

### FUNGICIDE PRODUCTION

A substrate for shake flasks was prepared with the following ingredients per litre: 4.0 g yeast extract, 1.0 g potassium dihydrogen phosphate, 0.1 g magnesium sulphate heptahydrate, 15 g glucose and 0.1 g Pluronic™ L61 (BASF, Federal Republic of Germany) and demineralized water. Sterilization took lace at 121°C for 20 minutes. A 500 ml Erlenmeyer flask with 100 ml of substrate was inoculated with 10⁶ spores from a YPG agar slant previously inoculated with CBS 616.87. The flasks were shaken at 230 rpm at 25°C for 3-7 days whereafter the fermentation broth was centrifuged. The supernatant containing the fungicide was thereby separated from the mycelium. The mycelium was discarded and the supernatant was analysed for fungicidal activity.

The fungicide can also be produced in surface cultures.

### ANALYSIS

10⁶ spores of Botrytis cinerea were added to 50 ml of dilute salts

| | | |
|---|---|---|
| ammonium hydrogen phosphate | 66 | mg |
| potassium dihydrogen phosphate | 68 | - |
| dipotassium hydrogen phosphate | 87 | - |
| calcium chloride-2-hydrate | 7.4 | - |
| magnesium chloride-6-hydrate | 10 | - |
| made up to 1 litre with distilled water and sterilized at 121°C for 20 minutes. | | |

This was mixed with 50 ml YPG agar at a temperature which favoured the viability of Botrytis spores suitably in the temperature range from about 30°C to about 45°C where the agar is kept fluid without any harm to the spores. 12 ml of this mixture were poured in 9 cm petri dishes and allowed to solidify. 1-5 holes of 4 mm diameter were punched in the agar and 15 »l culture broth was put in each hole.

The petri dishes were incubated at 20-25°C for 2 days. The presence of a fungicide will reveal itself as a clear non-growth zone around the holes - the larger the zone the stronger the fungicide. The supernatant under these conditions produced a clear zone of 25 mm.

### EXTRACTION OF THE ACTIVE PRINCIPLE

The active principle of the invention could be extracted by adsorption on Amberlite XAD-4 adsorbent which has been thoroughly washed with acetone, ethanol and distilled water. Application of the sample on the adsorbent is followed by several washings with distilled water. Elution is carried out with ethanol and acetone, and the active principle concentrated in vacuo at room temperature.

### PROTECTION OF GRAPES AGAINST BOTRYTIS CINEREA

Commercially available green grapes were singled off, washed and dried. The pedicels were retained on the fruits. Two holes, 1 mm deep, were made on the fruits with the points of a syringe containing 10⁶ /ml of Botrytis spores. The grapes were then incubated at room temperature in a humid atmosphere for 24 hours. They were then inundated in the culture broth and returned to the humid conditions for about 1 week. In another case, the fruits were submerged in the culture broth and 24 hours later they were pricked with a syringe containing Botrytis spares. Here, too, they were incubated in a humid atmosphere for about 1 week.

The results of this test are shown in table 1 below.

**Table 1**

| Type of Treatment | | % Protection | | |
|---|---|---|---|---|
| 1st day | 2nd day | Expt 1 | Expt 2 | *Expt 3 |
| Botrytis cinerea | Water | 0 | 0 | 0 |
| - - | Culture broth | 66 | 83 | 33 |
| Water | Botrytis cinerea | 0 | 0 | 0 |
| Culture broth | - - | 66 | 33 | 66 |

| | | | | |
|---|---|---|---|---|
| * In this experiment a strain of Botrytis cinerea which was resistant to 100 ppm Iprodione (Rhone Poulenc) was used. | | | | |

As can be seen in Table 1 the grapes which were treated with culture broth of CBS 616.87 were very effectively controlled against Botrytis cinerea when compared with those treated with water. An even higher degree of protection can be expected if the spores are not injected into the grapes. Also, the protection was obvious when the Botrytis cinerea was resistant to known chemical fungicides.

### FIELD TRIALS FOR PROTECTION AGAINST BOTRYTIS CINEREA

### I: Tomato

The fungicide was tested for is ability to protect tomato plants against Botrytis cinerea in green houses. Both the controls and the experimentals consisted of 3 plots each of 8 plants measuring 75-90 cm in height. 100 sores were made on the leaves of the 8 plants and they were immediately sprayed to run off with 1% of a freeze-dried sample of the culture broth. The controls were sprayed with water. Four (4) hours later each plant was sprayed with 4x10⁶ spores of Botrytis cinerea. The green house was kept at a temperature of 18-20° C and a relative humidity of about 90% for 2 weeks after which time the number of infected leaves were counted.

The control plants had a total of 126 infections while those treated with Cercospora fungicide had 44.

### II: Peas

20% eluate (see extraction of the active principle) was sprayed on peas (type Bodil) in the field to see if the plants could be protected against natural infection of Botrytis. Four (4) plots each of 30 sq. meters were used for the test and 4 as control. The following compounds were added to the eluate:
0.2% Bevaloid 211, a dispersing agent, (Bevaloid Ltd., P.O. Box 3, Flemingate, Berverley, North Humberside HK 270 NW, England),
1% alcopol, a surfactant (Allied Colloids Ltd., P.O. Box 38, Low Moor, Bradford, Yorkshire BD 120 JZ, England), and
1:1600 of Chevron Spray sticker, ortho product 2786.

The first spraying was done at the onset of flowering and the second 10 days later. When the pods were fully mature and the plants had dried down 10 plants were chosen at random from each plot and checked in the laboratory for Botrytis infection on the pods and stems. The results below show the average infection per plot.

| TREATMENT | % INFECTION | |
|---|---|---|
| | PODS | STEMS |
| Control | 11 | 63 |
| CBS 616.87 fungicide | 6 | 35 |

### COMBINATION WITH HERBICIDES

Petri dishes were prepared as described under "Analysis" and used to investigate the effect of herbicides on the Phaeoramularia fungicide. It was found that the fungicide was not affected when combined with Chlorsulfuron (du Pont) (0.05-0.5 mg/ml), Atrazine (Ciba Geigy) (1-10 mg/ml) and Simazine (Ciba Geigy) (0.1-1 mg/ml). Its activity was slightly increased (10-20%) when 0.1-10 mg/ml of Lasso (Monsanto) was added. The 4 herbicides had no effect on Botrytis when applied singly.

The fungicide may be used in cases where Botrytis cinerea is resistant to the chemicals which are used today. It can be used on fruits and vegetables e.g. grapes, strawberries, tomatoes, citrus fruits and for post harvest protection e.g. on carrots. It may also be used protective or curative on plants which are normally attacked by Botrytis.

### EFFECT OF THE FUNGICIDE ON DERMATOPHYTES

The fungicide produced by CBS 616.87 was tested on Trichophyton rubrum, T. mentagrophytes and Microsporum canis. These were recent clinical isolates and were cultivated at 25°C on agar containing the following ingredients in grams per liter:
Bacto Yeast Morphology agar 35, (NH₄)₂SO₄ 1.5, NaH₂P0₄.2H₂0 0.429, K₂HP0₄ 0.92, 5N Na0H 1 ml. - pH was 7.1. This substrate was sterilized at 121°C for 20 minutes.

Mycelia from the 3 fungi were disintegrated with sterile glass beads in sterile water and 1 drop applied to the centre of petri dishes containing the same agar as above but to which was added Phaeoramularia fungicide. The fungicide was added at a concentration of from 0.5 »g/ml to 5»g/ml of the eluate described under "Extraction of the active principle". Griseofulvin was used in comparison. Controls were prepared with the same agar but devoid of fungicide. Five (5) replicates were made for each test.

All the dishes were incubated at 25° for 5 days after which time they were observed for fungal growth.

### RESULTS

The Phaeoramularia fungicide totally prevented the growth of the 3 fungi at all the concentrations tested. That means the MIC was 0.5 »g/ml. The Minimum Inhibitory Concentration, MIC, for griseofulvin is 1.5 »g/ml, 3 »g/ml and 10 »g/ml for Trichophyton rubrum, Microsporum canis and T. mentagrophytes respectively.

### PRESERVATION OF WOOD

Poria placenta and Gloeophyllum trabeum, 2 wood inhabiting fungi, were cultivated on malt agar at 25°C for 4 days. Filter paper discs of 8 mm diameter were wetted with 25 »l eluate containing the fungicidal principle of the invention and placed on the agar at 20 mm from the edge of the actively growing colonies. There were 3 replicates. Further incubation showed that growth of the 2 fungi was strongly inhibited by the fungicide.

## Claims (Claims for the following Contracting State(s): BE, CH, DE, FR, GB, GR, IT, LI, LU, NL, SE)

1. A fungicidal active principle obtainable by cultivation of Phaeoramularia fusimaculans (Atk) Liu & Guo CBS 616.87.

2. A fungicidal composition comprising an effective amount of a fungicidally active principle obtainable by cultivation of Phaeoramularia fusimaculans (Atk) Liu & Guo CBS 616.87 and an agrochemically and/or physiologically compatible carrier or diluent.

3. A fungicidal composition according to Claim 2, wherein said fungicidally active principle is combined with one or more other active materials.

4. A fungicidal composition according to Claim 3, wherein said other active materials are chosen among biocides, pesticides, herbicides, insecticides, nematocides, acaricides and fungicides.

5. A fungicidal composition according to Claim 3, wherein said other active materials are chosen among plant nutrients, plant growth promoters and fertilizers.

6. A fungicidal composition according to Claim 3, wherein said other active materials are chosen among antibiotica and sulfonamides.

7. A fungicidal composition comprising an effective number of viable units (spores and/or mycelium) of a fungus of Phaeoramularia fusimaculans (Atk) Liu & Guo or a mutant or genetically engineered transformant having the same characteristic ability to produce said active principle suspended in a compatible medium.

8. A fungicidal composition according to Claim 7, wherein said fungus is the strain Phaeoramularia fusimaculans (Atk) Liu & Guo CBS 616.87, or a mutant or genetically engineered transformant having the same characteristic ability to produce said active principle.

9. Composition for use as a fungicide on animals including mammals, containing an effective amount of a fungicidally active principle obtainable by cultivation of Phaeoramularia fusimaculans (Atk) Liu & Guo CBS 616.87

10. A method of combating fungi in plants, wherein an effective amount of a fungicidal composition according to any of Claims 2 to 8 is applied to a region to be treated.

11. A method according to Claim 10, wherein the fungi to be combated belong to the genera Botrytis, Rhizomucor, Fusarium, Pyricularia, Penicillium and/or Rhizopus.

12. A method according to Claim 11, wherein said fungus is Botrytis.

13. A method according to Claim 12, wherein said Botrytis is Botrytis cinerea.

14. A process for the production of a fungicidally active principle, wherein Phaeoramularia fusimaculans (Atk) Liu & Guo or a mutant or genetically engineered transformant having the same characteristic ability to produce said active principle is cultivated in the presence of sources of assimilable nitrogen, carbon, and oxygen together with essential nutrients, and said active principle is recovered.

15. A process according to Claim 14, wherein said active principle is recovered from the culture broth.

16. A process according to Claim 15, wherein said active principle is recovered by subjecting said culture broth to adsorption chromatography and subsequent vacuum concentration.

17. A process according to Claim 14, 15, or 16, wherein said fungus is Phaeoramularia fusimaculans (Atk) Liu & Guo CBS 616.87, or a mutant or genetically engineered transformant having the same characteristic ability to produce said active principle.

18. The fungus Cercospora fusimaculans Atk deposited with Centraalbureau voor Schimmelcultures on October 22, 1987, and accorded the accession No. CBS 616.87, or a mutant or genetically engineered transformant having the same characteristic ability to produce said active principle.

19. A fungicidal growth medium additive comprising the fungicidal principle according to any of the claims or produced by the method of any one of Claims 14 to 17.

20. A fungicidal food additive comprising the fungicidal principle according to any of the claims or produced by the method of any one of Claims 14 to 17.

21. A fungicidal wood preservation additive comprising the fungicidal principle according to any of the claims or produced by the method of any one of Claims 14 to 17.

22. A fungicidal active principle obtainable by cultivation of Phaeoramularia fusimaculans (Atk) Liu & Guo CBS 616.87 for use in combating fungi.

23. Use of a fungicidal active principle obtainable by cultivation of Phaeoramularia fusimaculans (Atk) Liu & Guo CBS 616.87 for the manufacture of a medicament for combating fungi.

## Claims (Claims for the following Contracting State(s): AT, ES)

1. A process for the production of a fungicidally active principle, wherein *Phaeoramularia fusimaculans (Atk*) Liu & Guo or a mutant or genetically engineered transformant having the same characteristic ability to produce said active principle is cultivated in the presence of sources of assimilable nitrogen, carbon, and oxygen together with essential nutrients, and said active principle is recovered.

2. A process according to Claim 1, wherein said active principle is recovered from the culture broth.

3. A process according to Claim 2, wherein said active principle is recovered by subjecting said culture broth to adsorption chromatography and subsequent vacuum concentration.

4. A process according to Claim 1, 2, or 3, wherein said fungus is *Phaeoramularia fusimaculans (Atk*) Liu & Guo CBS 616.87, or a mutant or genetically engineered transformant having the same characteristic ability to produce said active principle.

5. A process for the production of a fungicidal composition comprising an effective amount of a fungicidally active principle produced in accordance to any of the claims 1 to 4, whereby said fungicidally active principle is mixed with an agrochemically and/or physiologically compatible carrier or diluent.

6. A process according to claim 5, wherein said fungicidally active principle is combined with one or more other active materials.

7. A process according to claim 6, wherein said other active materials are chosen among biocides, pesticides, herbicides, insecticides, nematocides, acaricides and fungicides.

8. A process according to claim 6, wherein said other active materials are chosen among plant nutrients, plant growth promoters and fertilizers.

9. A process according to claim 6, wherein said other active materials are chosen among antibiotica and sulfonamides.

10. A process for the production of a fungicidal composition comprising an effective number of viable units (spores and/or mycelium) of a fungus of *Phaeoramularia fusimaculans Atk*) Liu & Guo or a mutant or genetically engineered transformant having the same characteristic ability to produce said active principle, wherein said viable units are suspended in a compatible medium.

11. A process according to claim 10, wherein said fungus is the strain *Phaeoramularia fusimaculans (Atk*) Liu & Guo CBS 616.87, or a mutant or genetically engineered transformant having the same characteristic ability to produce said active principle.

12. A process for the production of a composition for use as a fungicide on animals including mammals, containing an effective amount of a fungicidally active principle obtainable by cultivation of *Phaeoramularia fusimaculans (Atk*) Liu & Guo CBS 616.87 which is mixed with a physiologically compatible carrier or diluent.

13. A method of combating fungi in plants, wherein an effective amount of a fungicidal composition produced according to any of Claims 5 to 11 is applied to a region to be treated.

14. A method according to Claim 13, wherein the fungi to be combated belong to the genera Botrytis, Rhizomucor, Fusarium, Pyricularia, Penicillium and/or Rhizopus.

15. A method according to Claim 14, wherein said fungus is Botrytis.

16. A method according to Claim 15, wherein said Botrytis is Botrytis cinerea.

17. The fungus *Cercospora fusimaculans Atk* deposited with Centraalbureau voor Schimmelcultures on October 22, 1987, and accorded the accession No. CBS 616.87, or a mutant or genetically engineered transformant having the same characteristic ability to produce said active principle.

18. A fungicidal growth medium additive comprising the fungicidal principle produced by a method of any one of Claims 1 to 4.

19. A fungicidal food additive comprising the fungicidal principle produced by a method of any one of Claims 1 to 4.

20. A fungicidal wood preservation additive comprising the fungicidal principle produced by a method of any one of Claims 1 to 4.

21. Use of a fungicidal active principle obtainable by cultivation of *Phaeoramularia fusimaculans (Atk*) Liu & Guo CBS 616.87 for the manufacture of a medicament for combating fungi.

## Patentansprüche (Patentansprüche für folgende(n) Vertragsstaat(en): BE, CH, DE, FR, GB, GR, IT, LI, LU, NL, SE)

1. Fungizides aktives Prinzip, erhältlich durch Kultivierung von Phaeoramularia fusimaculans (Atk) Liu & Guo CBS 616.87.

2. Fungizide Zusammensetzung, enthaltend eine wirksame Menge eines fungiziden aktiven Prinzips, das durch Kultivierung von Phaeoramularia fusimaculans (Atk) Liu & Guo CBS 616.87 erhältlich ist, und einen agrochemisch und/oder physiologisch verträglichen Träger oder Verdünnungsmittel.

3. Fungizide Zusammensetzung nach Anspruch 2, wobei das fungizide aktive Prinzip mit einem oder mehreren anderen aktiven Stoffen kombiniert ist.

4. Fungizide Zusammensetzung nach Anspruch 3, wobei die anderen aktiven Stoffe aus Bioziden, Pestiziden, Herbiziden, Insektiziden, Nematoziden, Akariziden und Fungiziden ausgewählt sind.

5. Fungizide Zusammensetzung nach Anspruch 3, wobei die anderen aktiven Stoffe aus Pflanzennährstoffen, das Pflanzenwachstum fördernden Substanzen und Düngemitteln ausgewählt sind.

6. Fungizide Zusammensetzung nach Anspruch 3, wobei die anderen aktiven Stoffe aus Antibiotika und Sulfonamiden ausgewählt sind.

7. Fungizide Zusammensetzung, enthaltend eine wirksame Anzahl lebensfähiger Einheiten (Sporen und/oder Mycelien) eines Pilzes von Phaeoramularia fusimaculans (Atk) Liu & Guo oder einer Mutante oder genetisch veränderten Transformante, die die gleiche charakteristische Fähigkeit zum Bilden des aktiven Prinzips besitzt, und die in einem passenden Medium suspendiert ist.

8. Fungizide Zusammensetzung nach Anspruch 7, wobei der Pilz der Stamm Phaeoramularia fusimaculans (Atk) Liu & Guo CBS 616.87 oder eine Mutante oder genetisch veränderte Transformante, die die gleiche charakteristische Fähigkeit zum Bilden des aktiven Prinzips besitzt, ist.

9. Zusammensetzung zur Verwendung als Fungizid an Tieren einschließlich Säugetieren, das eine wirksame Menge eines fungiziden aktiven Prinzips enthält, das durch Kultivierung von Phaeoramularia fusimaculans (Atk) Liu & Guo CBS 616.87 erhältlich ist.

10. Verfahren zum Bekämpfen von Pilzen bei Pflanzen, wobei eine wirksame Menge einer fungiziden Zusammensetzung nach einem der Ansprüche 2 bis 8 auf eine zu behandelnde Region aufgebracht wird.

11. Verfahren nach Anspruch 10, wobei die zu bekämpfenden Pilze zum Genus Botrytis, Rhizomucor, Fusarium, Pyricularia, Penicillium und/oder Rhizopus gehören.

12. Verfahren nach Anspruch 11, wobei der Pilz Botrytis ist.

13. Verfahren nach Anspruch 12, wobei der Botrytis Botrytis cinerea ist.

14. Verfahren zur Herstellung eines fungiziden aktiven Prinzips, wobei Phaeoramularia fusimaculans (Atk) Liu & Guo oder eine Mutante oder genetisch veränderte Transformante, die die gleiche charakteristische Fähigkeit zum Bilden des aktiven Prinzips besitzt, in Gegenwart von Quellen assimilierbaren Stickstoffs, Kohlenstoffs und Sauerstoffs zusammen mit essentiellen Nährstoffen kultiviert wird, und wobei das aktive Prinzip gewonnen wird.

15. Verfahren nach Anspruch 14, wobei das aktive Prinzip aus dem Kulturmedium gewonnen wird.

16. Verfahren nach Anspruch 15, wobei das aktive Prinzip gewonnen wird, indem das Kulturmedium einer Adsorptionschromatographie und einer anschließenden Vakuumkonzentration unterzogen wird.

17. Verfahren nach Anspruch 14, 15 oder 16, wobei der Pilz Phaeoramularia fusimaculans (Atk) Liu & Guo CBS 616.87 ist oder eine Mutante oder genetisch veränderte Transformante, die die gleiche charakteristische Fähigkeit zum Bilden des aktiven Prinzips besitzt.

18. Pilz Cercospora fusimaculans Atk, der bei dem Centraalbureau voor Schimmelcultures am 22. Oktober 1987 hinterlegt wurde und dem die Hinterlegungsnummer CBS 616.87 zugeteilt wurde, oder eine Mutante oder genetisch veränderte Transformante, die die gleiche charakteristische Fähigkeit zum Bilden des aktiven Prinzips besitzt.

19. Fungizider Wachstumsmediumzusatz, der das fungizide Prinzip nach einem der vorhergehenden Ansprüche oder hergestellt nach dem Verfahren nach einem der Ansprüche 14 - 17 aufweist.

20. Fungizider Nahrungsmittelzusatz, der das fungizide Prinzip nach einem der vorhergehenden Ansprüche oder hergestellt nach dem Verfahren nach einem der Ansprüche 14 - 17 aufweist.

21. Fungizider Holzkonservierungszusatz, der das fungizide Prinzip nach einem der vorhergehenden Ansprüche oder hergestellt nach dem Verfahren nach einem der Ansprüche 14 - 17 aufweist.

22. Fungizides aktives Prinzip, das durch Kultivierung von Phaeoramularia fusimaculans (Atk) Liu & Guo CBS 616.87 erhältlich ist, zur Verwendung bei der Bekämpfung von Pilzen.

23. Verwendung eines fungiziden aktiven Prinzips, das durch Kultivierung von Phaeoramularia fusimaculans (Atk) Liu & Guo CBS 616.87 erhältlich ist, zur Herstellung eines Medikamentes zur Bekämpfung von Pilzen.

## Patentansprüche (Patentansprüche für folgende(n) Vertragsstaat(en): AT, ES)

1. Verfahren zur Herstellung eines fungiziden aktiven Prinzips, wobei Phaeoramularia fusimaculans (Atk) Liu & Guo oder eine Mutante oder genetisch veränderte Transformante, die die gleiche charakteristische Fähigkeit zum Bilden des aktiven Prinzips besitzt, in Gegenwart von Quellen assimilierbaren Stickstoffs, Kohlenstoffs und Sauerstoffs zusammen mit essentiellen Nährstoffen kultiviert wird, und wobei das aktive Prinzip gewonnen wird.

2. Verfahren nach Anspruch 1, wobei das aktive Prinzip aus dem Kulturmedium gewonnen wird.

3. Verfahren nach Anspruch 2, wobei das aktive Prinzip gewonnen wird, indem das Kulturmedium einer Adsorptionschromatographie und einer anschließenden Vakuumkonzentration unterzogen wird.

4. Verfahren nach Anspruch 1, 2 oder 3, wobei der Pilz Phaeoramularia fusimaculans (Atk) Liu & Guo CBS 616.87 ist oder eine Mutante oder genetisch veränderte Transformante, die die gleiche charakteristische Fähigkeit zum Bilden des aktiven Prinzips besitzt.

5. Verfahren zur Herstellung einer fungiziden Zusammensetzung, die eine wirksame Menge eines nach einem der Ansprüche 1 - 4 hergestellten fungiziden aktiven Prinzips aufweist, wobei das fungizide aktive Prinzip mit einem agrochemisch und/oder physiologisch verträglichen Träger oder Verdünnungsmittel vermischt wird.

6. Verfahren nach Anspruch 5, wobei das fungizide aktive Prinzip mit einem oder mehreren anderen aktiven Stoffen kombiniert wird.

7. Verfahren nach Anspruch 6, wobei die anderen aktiven Stoffe aus Bioziden, Pestiziden, Herbiziden, Insektiziden, Nematoziden, Akariziden und Fungiziden ausgewählt werden.

8. Verfahren nach Anspruch 6, wobei die anderen aktiven Stoffe aus Pflanzennährstoffen, das Pflanzenwachstum fördernden Substanzen und Düngemitteln ausgewählt werden.

9. Verfahren nach Anspruch 6, wobei die anderen aktiven Stoffe aus Antibiotika und Sulfonamiden ausgewählt werden.

10. Verfahren zur Herstellung einer fungiziden Zusammensetzung, die aufweist: eine wirksame Anzahl lebensfähiger Einheiten (Sporen und/oder Mycelien) eines Pilzes von Phaeoramularia fusimaculans (Atk) Liu & Guo oder einer Mutante oder genetisch veränderten Transformante, die die gleiche charakteristische Fähigkeit zum Bilden des aktiven Prinzips besitzt, wobei die lebensfähigen Einheiten in einem passenden Medium suspendiert sind.

11. Verfahren nach Anspruch 10, wobei der Pilz der Stamm Phaeoramularia fusimaculans (Atk) Liu & Guo CBS 616.87 oder eine Mutante oder genetisch veränderte Transformante, die die gleiche charakteristische Fähigkeit zum Bilden des aktiven Prinzips besitzt, ist.

12. Verfahren zur Herstellung einer Zusammensetzung zur Verwendung als Fungizid an Tieren einschließlich Säugetieren, die eine wirksame Menge eines fungiziden aktiven Prinzips enthält, das durch Kultivierung von Phaeoramularia fusimaculans (Atk) Liu & Guo CBS 616.87 erhältlich ist, und das mit einem physiologisch verträglichen Träger oder Verdünnungsmittel vermischt wird.

13. Verfahren zur Bekämpfung von Pilzen bei Pflanzen, wobei eine wirksame Menge einer fungiziden Zusammensetzung nach einem der Ansprüche 5 bis 11 auf eine zu behandelnde Region aufgebracht wird.

14. Verfahren nach Anspruch 13, wobei die zu bekämpfenden Pilze zum Genus Botrytis, Rhizomucor, Fusarium, Pyricularia, Penicillium und/oder Rhizopus gehören.

15. Verfahren nach Anspruch 14, wobei der Pilz Botrytis ist.

16. Verfahren nach Anspruch 15, wobei der Botrytis Botrytis cinerea ist.

17. Pilz Cercospora fusimaculans Atk, der bei dem Centraalbureau voor Schimmelcultures am 22. Oktober 1987 hinterlegt wurde und dem die Hinterlegungsnummer CBS 616.87 zugeteilt wurde, oder eine Mutante oder genetisch veränderte Transformante, die die gleiche charakteristische Fähigkeit zum Bilden des aktiven Prinzips besitzt.

18. Fungizider Wachstumsmediumzusatz, der das fungizide Prinzip, das nach einem Verfahren nach einem der Ansprüche 1 - 4 hergestellt wurde, aufweist.

19. Fungizider Nahrungsmittelzusatz, der das fungizide Prinzip, das nach einem Verfahren nach einem der Ansprüche 1 - 4 hergestellt wurde, aufweist.

20. Fungizider Holzkonservierungszusatz, der das fungizide Prinzip, das nach einem Verfahren nach einem der Ansprüche 1 - 4 hergestellt wurde, aufweist.

21. Verwendung eines fungiziden aktiven Prinzips, das durch Kultivierung von Phaeoramularia fusimaculans (Atk) Liu & Guo CBS 616.87 erhältlich ist, zur Herstellung eines Medikaments zur Bekämpfung von Pilzen.

## Revendications (Revendications pour l'(les) Etat(s) contractant(s) suivant(s): BE, CH, DE, FR, GB, GR, IT, LI, LU, NL, SE)

1. Un principe actif fongicide qui peut être obtenu par culture de *Phæoramularia fusimaculans (Atk*) Liu & Guo CBS 616.87.

2. Une composition fongicide comprenant une quantité efficace d'un principe fongicidement actif qui peut être obtenu par culture de *Phæoramularia fusimaculans (Atk*) Liu & Guo CBS 616.87 et un véhicule ou diluant agrochimiquement et/ou physiologiquement compatible.

3. Une composition fongicide selon la revendication 2, dans laquelle le principe fongicidement actif est combiné à une ou plusieurs autres matières actives.

4. Une composition fongicide selon la revendication 3, dans laquelle les autres matières actives sont choisies parmi les biocides, les pesticides, les herbicides, les insecticides, les nématocides, les acaricides et les fongicides.

5. Une composition fongicide selon la revendication 3, dans laquelle les autres matières actives sont choisies parmi les substances nutritives des végétaux, les promoteurs de la croissance des végétaux et les engrais.

6. Une composition fongicide selon la revendication 3, dans laquelle les autres matières actives sont choisies parmi les antibiotiques et les sulfamides.

7. Une composition fongicide comprenant un nombre efficace d'unités viables (spores et/ou mycelium) d'un fungus de *Phæoramularia fusimaculans (Atk)* Liu & Guo ou d'un mutant ou d'un transformant obtenu par génie génétique possédant la même aptitude caractéristique à produire ledit principe, en suspension dans un milieu compatible.

8. Une composition fongicide selon la revendication 7, dans lequel le fungus est la souche *Phæoramularia fusimaculans (Atk*) Liu & Guo CBS 616.87, ou un mutant ou transformant obtenu par génie génétique possédant la même aptitude caractéristique à produire ledit principe actif.

9. Composition pour une utilisation comme fongicide sur des animaux incluant les mammifères, contenant une quantité efficace d'un principe fongicidement actif pouvant être obtenu par culture de *Phæoramularia fusimaculans (Atk)* Liu & Guo CBS 616.87.

10. Un procédé pour combattre les fungi dans les végétaux, dans lequel on applique à une région à traiter une quantité efficace d'une composition fongicide selon l'une quelconque des revendications 2 à 8.

11. Un procédé selon la revendication 10, dans lequel les fungi à combattre appartiennent aux genres Botrytis, Rhizomucor, Fusarium, Pyricularia, Penicillium et/ou Rhizopus.

12. Un procédé selon la revendication 11, dans lequel le fungus est Botrytis.

13. Un procédé selon la revendication 12, dans lequel le Botrytis est Botrytis cinerea.

14. Un processus de production d'un principe fongicidement actif, dans lequel on cultive *Phæoramularia fusimaculans (Atk*) Liu & Guo, ou un mutant ou transformant obtenu par génie génétique possédant la même aptitude caractéristique à produire ledit principe actif, en présence de sources d'azote assimilable, de carbone et d'oxygène ainsi qu'avec des éléments nutritifs essentiels, et on récupère ledit principe actif.

15. Un processus selon la revendication 14, dans lequel le principe actif est récupéré du bouillon de culture.

16. Un processus selon la revendication 15, dans lequel le principe actif est récupéré en soumettant le bouillon de culture à une chromatographie par adsorption puis à une concentration sous vide.

17. Un processus selon la revendication 14, 15 ou 16, dans lequel le fungus est *Phæoramularia fusimaculans (Atk*) Liu & Guo CBS 616.87, ou un mutant ou transformant obtenu par génie génétique possédant la même aptitude caractéristique à produire ledit principe actif.

18. Le fungus Cercospora fusimaculans Atk déposé au Centraalbureau voor Schimmelcultures le 22 octobre 1987 et ayant reçu le numéro d'accession CBS 616.87, ou un mutant ou transformant obtenu par génie génétique possédant la même aptitude caractéristique à produire ledit principe actif.

19. Un additif fongicide pour milieu de croissance, comprenant le principe fongicide de l'une quelconque des revendications ou produit par le procédé de l'une quelconque des revendications 14 à 17.

20. Un additif fongicide alimentaire, comprenant le principe fongicide de l'une quelconque des revendications ou produit par le procédé de l'une quelconque des revendications 14 à 17.

21. Un additif fongicide conservateur du bois, comprenant le principe fongicide de l'une quelconque des revendications ou produit par le procédé de l'une quelconque des revendications 14 à 17.

22. Un principe actif fongicide qui peut être obtenu par culture de *Phæoramularia fusimaculans (Atk*) Liu & Guo CBS 616.87 destiné à être utilisé pour combattre les fungi.

23. L'utilisation d'un principe actif fongicide qui peut être obtenu par culture de *Phæoramularia fusimaculans (Atk*) Liu & Guo CBS 616.87 pour la fabrication d'un médicament pour combattre les fungi.

## Revendications (Revendications pour l'(les) Etat(s) contractant(s) suivant(s): AT, ES)

1. Un processus de production d'un principe fongicidement actif, dans lequel on cultive *Phæoramularia fusimaculans (Atk*) Liu & Guo, ou un mutant ou transformant obtenu par génie génétique possédant la même aptitude caractéristique à produire ledit principe actif, en présence de sources d'azote assimilable, de carbone et d'oxygène ainsi qu'avec des éléments nutritifs essentiels, et on récupère ledit principe actif.

2. Un processus selon la revendication 1, dans lequel le principe actif est récupéré du bouillon de culture.

3. Un processus selon la revendication 2, dans lequel le principe actif est récupéré en soumettant le bouillon de culture à une chromatographie par adsorption puis à une concentration sous vide.

4. Un processus selon la revendication 1, 2 ou 3, dans lequel le fungus est *Phæoramularia fusimaculans (Atk*) Liu & Guo CBS 616.87, ou un mutant ou transformant obtenu par génie génétique possédant la même aptitude caractéristique à produire ledit principe actif.

5. Un processus pour la production d'une composition fongicide comprenant une quantité efficace d'un principe fongicidement actif produit conformément à l'une quelconque des revendications 1 à 4, dans lequel le principe fongicidement actif est mélangé à un véhicule ou diluant agrochimiquement et/ou physiologiquement compatible.

6. Un processus selon la revendication 5, dans lequel le principe fongicidement actif est combiné à une ou plusieurs autres matières actives.

7. Un processus selon la revendication 6, dans lequel les autres matières actives sont choisies parmi les biocides, les pesticides, les herbicides, les insecticides, les nématocides, les acaricides et les fongicides.

8. Un processus selon la revendication 6, dans lequel les autres matières actives sont choisies parmi les substances nutritives des végétaux, les promoteurs de la croissance des végétaux et les engrais.

9. Un processus selon la revendication 6, dans lequel les autres matières actives sont choisies parmi les antibiotiques et les sulfamides.

10. Un processus pour la production d'une composition fongicide comprenant un nombre efficace d'unités viables (spores et/ou mycelium) d'un fungus de *Phæoramularia fusimaculans (Atk*) Liu & Guo ou d'un mutant ou d'un transformant obtenu par génie génétique possédant la même aptitude caractéristique à produire ledit principe, dans lequel les unités viables sont en suspension dans un milieu compatible.

11. Un processus selon la revendication 10, dans lequel le fungus est la souche *Phæoramularia fusimaculans (Atk*) Liu & Guo CBS 616.87, ou un mutant ou transformant obtenu par génie génétique possédant la même aptitude caractéristique à produire ledit principe actif.

12. Un processus pour la production d'une composition pour une utilisation comme fongicide sur des animaux incluant les mammifères, contenant une quantité efficace d'un principe fongicidement actif pouvant être obtenu par culture de *Phæoramularia fusimaculans (Atk*) Liu & Guo CBS 616.87 en mélange avec un diluant ou véhicule physiologiquement compatible.

13. Un procédé pour combattre les fungi dans les végétaux, dans lequel on applique à une région à traiter une quantité efficace d'une composition fongicide produite selon l'une quelconque des revendications 5 à 11.

14. Un procédé selon la revendication 13, dans lequel les fungi à combattre appartiennent aux genres Botrytis, Rhizomucor, Fusarium, Pyricularia, Penicillium et/ou Rhizopus.

15. Un procédé selon la revendication 14, dans lequel le fungus est Botrytis.

16. Un procédé selon la revendication 15, dans lequel le Botrytis est Botrytis cinerea.

17. Le fungus *Cercospora fusimaculans Atk* déposé au Centraalbureau voor Schimmelcultures le 22 octobre 1987 et ayant reçu le numéro d'accession CBS 616.87, ou un mutant ou transformant obtenu par génie génétique possédant la même aptitude caractéristique à produire ledit principe actif.

18. Un additif fongicide pour milieu de croissance, comprenant le principe fongicide produit par un procédé de l'une quelconque des revendications 1 à 4.

19. Un additif fongicide alimentaire, comprenant le principe fongicide produit par un procédé de l'une quelconque des revendications 1 à 4.

20. Un additif fongicide conservateur du bois, comprenant le principe fongicide produit par un procédé de l'une quelconque des revendications 1 à 4.

21. L'utilisation d'un principe actif fongicide qui peut être obtenu par culture de *Phæoramularia fusimaculans (Atk*) Liu & Guo CBS 616.87 pour la fabrication d'un médicament pour combattre les fungi.
